# EUROPEAN PATENT SPECIFICATION

(11) **EP 1 079 889 B1**
(45) Date of publication and mention of the grant of the patent: **03.08.2005**
(21) Application number: 99920299.7
(22) Date of filing: 03.05.1999
(51) Int. Cl.: A61M 37/00

(54) **GAS-DRIVEN SPRAYING OF MIXED SEALANT AGENTS**
GASGETRIEBENE SPRÜHVORRICHTUNG FÜR GEMISCHTE FIBRINKLEBER
PULVERISATION A ENTRAINEMENT PAR GAZ POUR MELANGE DE CICATRISANTS

(30) Priority: 01.05.1998 US 83854 P
(43) Date of publication of application: 07.03.2001
(73) Proprietor: BAXTER INTERNATIONAL INC., Deerfield, Illinois 60015 (US)
(72) Inventor: EPSTEIN, Gordon, H., Fremont, CA 94539 (US); LEVINSON, Mitchell, E., Pleasanton, CA 94588 (US)
(74) Representative: Baker, Colin John
(86) International application number: PCT/US1999/009663
(87) International publication number: WO 1999/056815

(56) References cited:
- WO-A-96/39212
- US-A- 4 846 405
- US-A- 5 116 315
- US-A- 5 163 433
- US-A- 5 474 540
- US-A- 5 582 596
- US-A- 5 665 067
- US-A- 5 759 169
- US-A- 5 759 171

## Description

### RELATED APPLICATIONS

This application discloses subject matter related to our copending International Patent Application Nos. PCT/US98/07488 and PCT/US98/07846 both filed April 14, 1998, PCT/US98/21045 filed 6 October 1998 and PCT/US99/04830 filed 5 March 1999 which applications are referred to as "the above applications".

### BACKGROUND OF THE INVENTION

### 1. Field of the Invention

The present invention relates to an applicator for applying multiple fluid sealant agents to a work surface and is particularly, although not exclusively, useful for applying tissue sealant agents to biological tissue to effect hemostasis or achieve other therapeutic results. More particularly, it rotates to spray application of tissue sealants from a hand-held applicator.

### 2. Description of Related Art

Use of tissue sealants and other biological materials is an important emerging surgical technique, well adapted for the operating room or field environments such as the doctor's office or mobile medical units. Preferred sealants include fibrin sealants which are formed from blood plasma components and comprise, on the one hand, a first agent containing fibrinogen and Factor XIII and on the other hand a second agent which usually includes thrombin, and calcium ions. The fibrinogen is capable of a polymerizing and being cross-linked to form a solid fibrin clot when the agents are mixed. The necessary additional factors to simulate relevant portions of the natural blood coagulation cascade are suitably distributed between the fibrinogen and thrombin agents.

High levels of protection against transmission of infections or induction of immunological reactions can be assured by using an autologous or single-donor source for both agents. Such sealants are highly effective, are biologically degraded without residue and may promote wound healing.

Depending upon the potency of the particular formulations employed, coagulation of the sealant may take place very rapidly, yielding a gel within perhaps 10 or 20 seconds. Though often very desirable for surgical reasons, such fast-acting properties present potential problems of fouling or clogging. These problems must be overcome in devising suitable applicators, and methods of application.

A popular manually operable applicator for such two-agent sealants employs a dual syringe construction wherein two syringes, connected by a yoke, each provide a reservoir for one of the agents. In most prior devices, the sealant agents are discharged in separate streams and mixed externally of the applicator. Such applicators are similar in principle to household epoxy glue applicators commonly available in hardware stores. Achieving effective mixing externally of the applicator is problematic.

In United States patent No. 5,266,877, and the above applications, the present inventor teaches various constructions of a dual syringe applicator wherein the fluid sealant agents are mixed internally.

In one or more of the above copending applications the possibility of retrograde clearing of the mixed fluids pathway within the applicator, using suction, is also disclosed. The applicator is provided with suitable suction conduits and valving to apply suction to the work surface, to prepare it for the application of sealant, for example by removing fluids. As taught, the valving is operable to effect retrograde clearing of a sealant dispensing pathway. Enhanced mixing results and problems of fouling by deposited solids are avoided.

Such devices are useful primarily for localized tissue treatment to close wounds and the like. More widespread events, such as diffuse oozing and sealing of tissues to prevent fluid leaks, are better treated by spray applicators and a number have been proposed with varying degrees of complexity and success.

Most prior proposals for spray applicators, including applicant's own invention disclosed in a patent application No. PCT/US99/04830 , abide by the principle of external mixing because of the difficulties inherent in internal mixing. The problem of discharging a rapidly coagulating stream of mixed sealant agents through the narrow valves and orifices typically employed in spray dispensers, while preventing the applicator clogging and becoming unusable, is imposing. The problem is compounded when it is realized that such applicators are intended for occasional use: they may be used in short bursts of a few seconds each, being then set down to be used again several minutes later, providing plenty of opportunity for residual sealant to clog the applicator.

Capozzi et al. U. S. Patent Number 5,116, 315 discloses a spray applicator that outputs a mixture of two sealant agents which applicator is provided with replaceable spray orifices so that when they become clogged with sealant, the user can remove the clogged orifice and replace it with a new one. Changing the clogged orifices is inconvenient and may be difficult and inconvenient during surgery. Fibrin sealants can coagulate very quickly, in a matter of seconds. Accordingly, such a spray applicator can easily become clogged between applications if it is set down for a minute or two. The need to change the spray orifices during a surgical procedure, and to have replacement orifices available, may be quite disruptive or impractical.

Furthermore, the momentum of the spray droplets leaving the applicator is derived from manually applied pressure to the sprayed liquid. Naturally, the pressure with which the liquid is applied to the spray orifice, and therefore the momentum with which the droplets are discharged from the spray orifice, is subject to variation, caused by the mechanics of the dispensing mechanism or simple inability of a human operator to apply constant pressure throughout a range of manual movement. Accordingly with the Capozzi spray applicators, it is difficult to produce an even spray having a consistent spray pattern. It is also difficult to discharge the droplets from the applicator with a constant momentum, as would be desirable for consistent application of materials such as a surgical sealant.

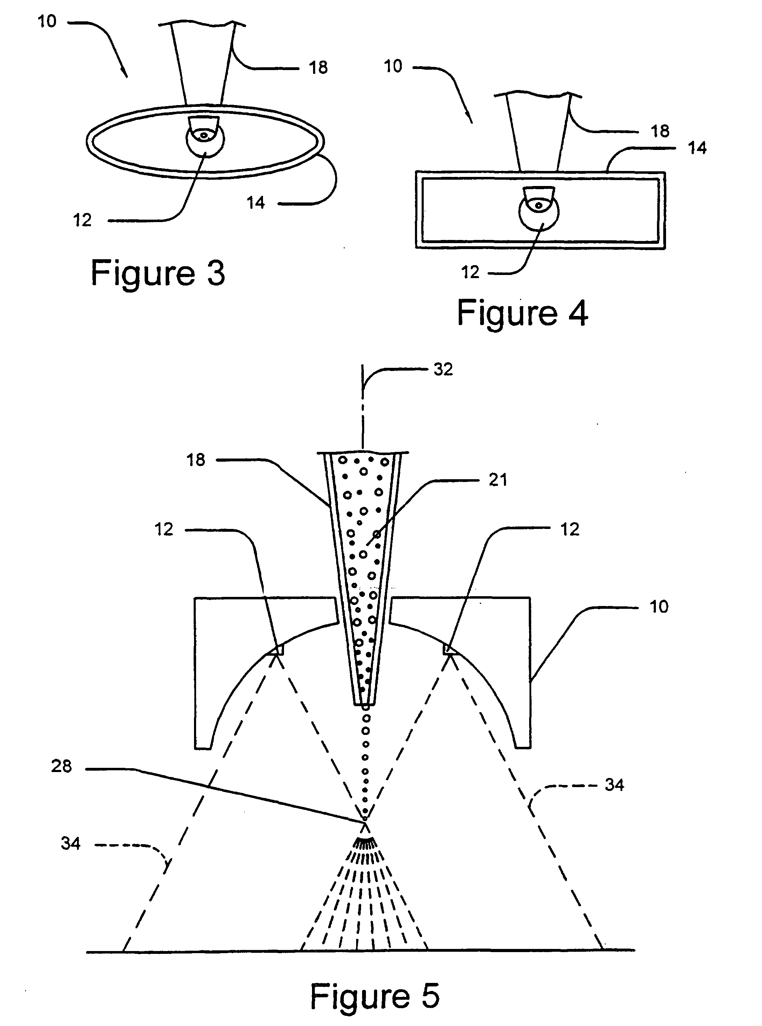

Prior to the present invention, a solution to this problem has been to apply the individual sealant agents in overlapping spray patterns, with the intention that the agents will mix in the air or on the tissue surface. Thus, for example, Redl US Patent 4,359,049 discloses a dual syringe tissue adhesive applicator for dispensing two adhesive agents, which applicator employs a disposable mixing needle to provide a mixed sealant output in the form of a liquid stream or a series of droplets. To provide a spray output, Figures 4 and 5 of Redl disclose the use of two pressurized gas streams emerging from outlets 50, 51 in the region of the valves 43, 44 of conveying channels 41, 40 which separately supply adhesive agents from syringe bodies 1 and 2. (See column 3, lines 45-47). Two spray patterns are generated and, according to Redl "unite at a distance of about 10 to 20 cm, rapidly forming a thin uniform adhesive film on the surface to be adhered or sealed.", see column 4 lines 1-3.

US-A-5,665,067 describes an apparatus for applying a multiple component tissue adhesive. Separate components are supplied from syringe bodies via component conveying channels to openings, where the components are entrained in a gas flow and mixed to provide a spray.

Pursuant to the present invention, it has been discovered that sealant agents discharged in separate sprays do not mix effectively. Apparently, the individual agents are frequently deposited on a work surface as individual or isolated drops or droplets that are unable to coalesce with droplets of the complementary agent. Thus mixing may be incomplete or, at best, slow. A further difficulty is that of maintaining a high degree of overlap of spatially separated sprays. Droplets deposited outside the zone of overlap are not combined with the complementary agent and therefore are not useful: valuable sealant product is wasted. Additionally, because the agents are not optimally mixed the resultant sealant may lack adhesive or cohesive strength.

There is accordingly a need for a sealant spray applicator, and method of applying a multi-agent sealant, which can effectively deliver multiple sealant agents to a work surface, for example a biological tissue, in an efficiently mixed state.

### SUMMARY OF THE INVENTION

According to the present invention, there is provided a spray applicator according to claim 1 and a spray tip assembly for use in a spray applicator according to claim 28.

The present invention solves the problem of providing a sealant spray applicator, which can effectively deliver multiple sealant agents to a work surface, for example a biological tissue, in an efficiently mixed state.

In one aspect, the invention provides a spray applicator for a liquid sealant comprising multiple sealant agents at least two of which are complementary one to the other and polymerize when mixed, the applicator comprising a sealant dispensing pathway terminating in a dispensing orifice through which the sealant agents are dispensed and a gas-entrainment chamber wherein mixed fluid sealant from the dispensing orifice is entrained in a gas stream for delivery to the work surface. Preferably a retrograde clearing device can be operated to apply suction to the sealant dispensing pathway, between applications of sealant, to remove clogs therefrom.

There is also described a method of spraying liquid sealant agents on to a work surface comprising mixing the liquid sealant agents in the liquid state and discharging the mixed liquid into a gas stream for dispersion of the liquid into droplets and delivery to the work surface. Optionally, the method can include a suction clearing step, between spray applications, to remove undesired deposits of mixed sealant.

By actively mixing the sealant agents prior to spraying ,the invention can assure that an effective sealant composition reaches the work surface and deposit of unmixed agents is avoided. Such active premixing is also helpful in initiating coagulation and thus providing a fast-acting sealant process. Furthermore, wasteful deposit on the work surface, of unmixed sealant agents, which will not coagulate into sealant, is avoided.

Use of a pressurized gas source to impel the spray facilitates generation of a constant velocity spray for even application of the sealant. It is difficult to obtain a steady spray by manually pressurizing of liquid sealant, as is required by conventional applicators such as those taught by Capozzi.

Preferably, although not necessarily, the sealant is a biological sealant, for example a tissue adhesive, and the work surface is a biological tissue subject to surgery. The sealant agents comprise a first, structural agent capable of gelling, and preferably of solidification and a second, activation agent which activates such gelling and, optionally, solidification. More preferably, the sealant is a tissue sealant and the first agent comprises fibrinogen and the second agent comprises, or can generate a fibrinogen activator, especially thrombin or an equivalent thereof.

### BRIEF DESCRIPTION OF THE DRAWINGS

One way of carrying out the invention is described in detail below with reference to the drawings which illustrate one or more specific embodiments of the invention and in which:-
- Figure I: a schematic sectional view of a sealant spray applicator according to the invention;
- Figure 2: is a view on the line 2 -2 of Figure 1;
- Figure 3: is a view on the line 3 -3 of Figure 1, to a reduced scale showing an alternative embodiment of spray applicator;
- Figure 4: is a view similar to Figure 3, showing another embodiment of spray applicator,
- Figure 5: is a view similar to Figure 1 showing a still further embodiment of spray applicator having multiple gas nozzles;
- Figure 6: is a partial perspective view, partially cut away, of a still further embodiment of spray applicator,
- Figure 7: is a side elevation of another embodiment of spray applicator according to the invention, depicted in relation to a work surface;
- Figure 8: is an exploded view of a spray tip assembly, showing the individual agents of the assembly, according to a further embodiment of the invention, which spray tip assembly is intended for use with an applicator body;
- Figure 9: is a cross-sectional view of the spray tip assembly of Figure 8, on a median plane, with the agents assembled together;
- Figure 10: is a plan view of a suction nose being a agent of a spray tip assembly shown in Figures 8 and 9;
- Figure 11: is a section on the line 11-11 of the suction nose shown in Figure 10;
- Figure 12: is a righthand end elevation of the suction nose shown in Figure 10;
- Figure 13: is an enlarged view of a detail of Figure 12;
- Figure 14: is a distal perspective view of the suction nose of Figure 10, to a reduced scale;
- Figure 15: is a proximal perspective view of the suction nose of Figure 10, to a reduced scale;
- Figure 16: is a distal perspective view of a spray nose plate being a agent of the spray tip assembly shown in Figure 8 and 9;
- Figure 17: is a proximal perspective view of the spray nose plate agent shown in Figure 16;
- Figure 18: is an underneath side perspective view from its proximal end of a spray nozzle being a agent of the spray tip assembly shown in Figures 8 and 9;
- Figure 19: is an underneath perspective view, from its distal end of the spray nozzle shown in Figure 18;
- Figure 20: is an end view, as seen in the proximal direction, of the spray nozzle shown in Figure 18;
- Figure 20A: is a schematic end view, as seen looking in a proximal direction, of an alternative embodiment wherein a spray nozzle is supplied with sealant from two lumens;
- Figure 21: is an end view, as seen in the distal direction, of the spray nozzle shown in Figure 18;
- Figure 22: is a plan view of the spray nozzle shown in Figure 18;
- Figure 23: is an underneath side perspective view from its distal end of an application tip being a agent of the spray tip assembly shown in Figures 8 and 9;
- Figure 24: is a cross-sectional view from the lefthand side on a center plane of the application tip shown in Figure 23;
- Figure 25: is a side perspective view from above, in the distal direction, of the application tip shown in Figure 23;
- Figure 26: is a side perspective view from its proximal end of a cover, being a agent of the spray tip assembly shown in Figures 8 and 9;
- Figure 27: is a side perspective view in the proximal direction, of the cover shown in Figure 26;
- Figure 28: is a cross-sectional view from the righthand side on a center plane of the cover shown in Figure 26;
- Figure 29: is an underneath perspective view of a manifold being a agent of the spray tip assembly shown in Figures 8 and 9;
- Figure 30: is a side perspective view, from above, of the manifold shown in Figure 29;
- Figure 31: is a side perspective view in the proximal direction of a vacuum tube assembly being a agent of the spray tip assembly shown in Figures 8 and 9;
- Figure 32: is an underneath perspective view, from one side, of the manifold shown in Figure 31;
- Figure 33: is a top perspective view of a shuttle valve being a agent of the spray tip assembly shown in Figures 8 and 9;
- Figure 34: is an underneath perspective view, of the shuttle valve shown in Figure 33;
- Figure 35: is a side elevational view, to a larger scale, of another embodiment of the invention comprising a gas-driven shear-spray device, wherein a liquid to be sprayed is impacted on a shearing plate and entrained in a gas stream; and
- Figure 36: is a side perspective view, looking in the proximal direction, of the shear-spray device of Figure 35.

### DESCRIPTION OF THE PREFERRED EMBODIMENTS

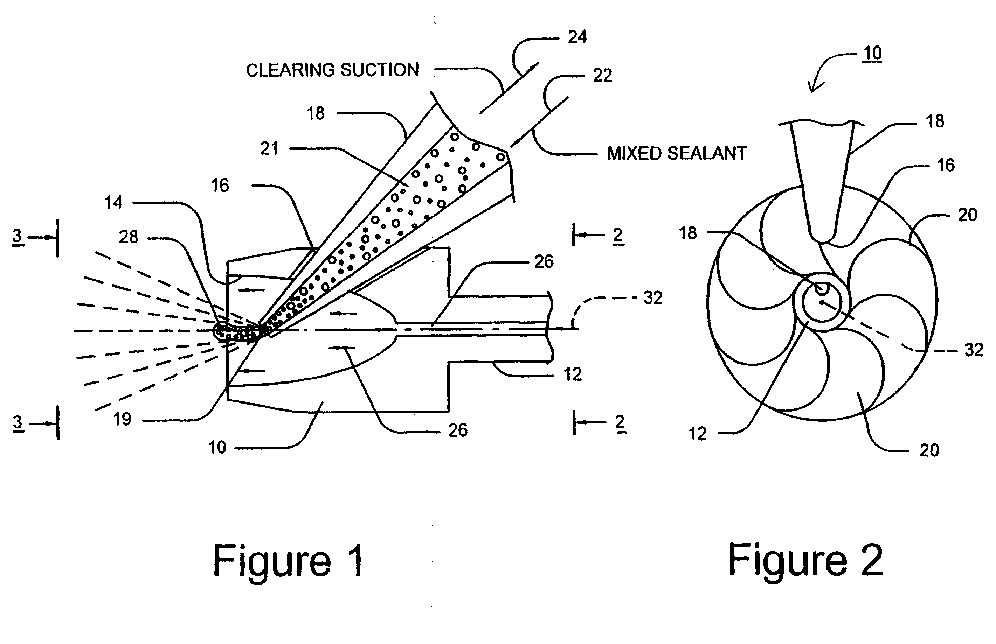

Referring to Figures 1 and 2 of the drawings, the sealant spray applicator shown comprises a spray hood 10 defining an outwardly diverging spray chamber. Spray hood 10 has a gas inlet 12, a spray discharge mouth 14 and an opening 16 in its upper wall through which extends a sealant dispensing lumen 18 terminating in a dispensing aperture 19. Dispensing aperture 19 is preferably positioned clear of the inner wall of hood 14, somewhat above the center of gas inlet 12, so that dispensed sealant 21 emerges into the heart of the gas stream. As shown in Figure 2, spray hood 10 can optionally be formed of relatively slidable overlapping vanes 20, giving it a variable configuration.

Dispensing lumen 18 can, for example, be the dispensing lumen, referenced 106, of an embodiment of non-spray applicator such as shown and described in co-pending international application No.

PCT/US99/04830 (Suction aspiration lumen 110 shown in that application is not employed in the embodiment of spray applicator invention described herein.)

The novel spray applicator described herein may be adapted also to apply suction to the work surface, for example, in a manner comparable with the teaching in application PCT/US99/04830 which employed a protruding suction nose 306 with an extensible end portion 314. Such a nose has particular utility for the present invention as a spacer to ensure that a minimum distance is maintained between the air stream from the sprayer and the patient tissue surface. This mitigates the well known risk of gas embolism which can be caused by fast-moving air too close to surgical wounds.

My prior application PCT/US99/04830 discloses various sealant applicators comprising an applicator body provided with a pair of syringe reservoirs, for two liquid sealant agents, which agents are dispelled from the syringe reservoirs by manual pressure applied to a trigger-like actuator. The body also is fitted with a suction connection and manually operable suction control valve. A range of applicator heads or tips is mountable on to the applicator body to provide selected functionality. A mixing tip (or head) enables the two liquid agents to be discharged into a mixing chamber where they are thoroughly mixed, and provides a flow of mixed sealant out of the applicator. This flow may be the source of mixed sealant 21 shown moving down lumen 18 in the direction of arrow 22, in Figure I herewith.

A preferred mixing tip also enables the operator to terminate sealant flow and apply suction to the dispensing lumen 106 or 18 in an opposite direction, shown by arrow 24 in accompanying Figure 1, to clear it of polymerized sealant and debris. The technique may be termed "retrograde" clearing. The sealant dispensing pathway can be appropriately configured, with a non-diminishing cross-section in the clearing direction, to facilitate such clearing. Such an applicator, provided with a mixing tip and suction clearing, is preferred in the practice of the present invention.

Pressurized gas, indicated by arrows 26, is admitted through inlet 12 and travels generally in the direction of arrows 26 to emerge through mouth 14 of spray hood 10. Sealant drops 28 emerging from lumen 18 are entrained in the gas flow 26 and disperse into droplets to emerge through mouth 14 in a well-defined spray 30.
Spray 30 can be applied to work surface, such as to biological tissues during animal or human surgery, by suitable positioning and movement of the spray applicator.

Because the spray droplets comprise sealant which has been thoroughly mixed prior to being dispersed into droplets, a high quality coating of sealant is deposited on the work surface in an efficient manner. Substantially all the sprayed sealant, if properly applied can be utilized, there being little or no possibility of unmixed agents being deposited on tissue. Depending upon the potency of its constituents, the sealant coagulates more or less rapidly, with good coverage and little wastage providing excellent control of sealant application. Controlling wastage is important because production of fibrin glue is expensive and, particularly in the case of a single donor or of autologous glue, only a few cc may be available.

Both the gas velocity and rate of sealant discharge are readily controlled, by suitable valves or other control structures (not shown), providing excellent management of the quality of the generated spray and -the characteristics of the sealant film deposited on the work surface. Thus, for example, a very fine mist, yielding an exceptionally thin sealant coating, can be generated by carefully dispensing a small drop of sealant 21 into a relatively fast moving gas flow. Faster dispensing of sealant 21, or slower gas velocity, or both, increases the size of the sprayed droplets yielding a thicker film of sealant at the work surface.

Because gas flow 26 carries emerging sealant away from lumen 18 while it is still liquid, and sweeps the exterior of the lumen, there is little risk of external fouling of lumen 18 by accumulations of coagulating sealant.

Additionally, it is desirable to avoid fouling mouth 14 with sealant-laden spray by suitably positioning aperture 19 and, possibly, by suitable choice of the dimensions of hood 10. The smoothly curved character of the exterior surface of lumen 18 or, more specifically, its conical or frusto-conical shape is believed to assist development of a desirable air flow and "atomization" or dispersion of the dispensed liquid into minute droplets. Unlike conventional sealant sprayers which rely upon pressurized discharge of sealant agents through one or more nozzles to impart momentum to the sprayed liquid, the spray device of the present invention is not subject to problems with drips and dribbles, which can be removed by the gas stream. Switching of the gas stream on and off should be suitably coordinated with discharge of sealant from lumen 18, or the gas may flow continuously during a surgical procedure while sealant spray is discharged in short bursts from time to time. Use of retrograde suction clearing removes any liquid sealant that could be a source of such drips from lumen 18, leaving it in pristine condition between uses.

When flow of mixed sealant 21 ceases, residual sealant in lumen 18 can coagulate and clog the applicator, if left in place. By means of the invention, employing the novel applicator disclosed in the above applications, the operator can apply suction in the direction of arrow 24 to clear lumen 18 of residual sealant. Preferably such suction application is effected promptly after ceasing delivery of sealant, while residual sealant in the lumen 18 is still liquid. However the downwardly tapering shape of lumen 18 facilitates removal of solid material should any be deposited.

Alternatively, lumen 18 and any other agent contacting mixed sealant could be rendered disposable. However such disposability is less desirable than retrograde suction clearing, is inconvenient and is not compatible with frequent use of the spray applicator of the invention in a busy, stressful surgical environment.

The spray applicator of Figure 1 provides a single coherent, pattern of spray comprising a good dispersion of well-mixed sealant droplets.

While the invention is not limited to the particular configuration shown in the drawing, and other suitable constructions will be apparent those skilled in the art, it may be seen from Figures 1-4 that dispensing aperture 19 of lumen 18 is disposed in the downstream half of spray chamber 11, approximately on a central axis 32 of spray chamber 11, (Figure 1) or a little above central axis 32, to allow for the effects of gravity (Figures 2-4). Also aperture 19 is preferably positioned upstream of mouth 14, and does not project through mouth 14, to encourage "atomization" or good dispersion of the sealant liquid into small droplets. Furthermore, lumen 18 is preferably disposed at an angle to the direction of flow of gas stream 26 such that emerging sealant drops 28 will have a significant agent of movement in that direction and will be drawn away from lumen 18 by the gas flow. Thus, in the preferred embodiment shown in Figure 1, lumen 18 is disposed to extend in the direction of gas flow 26 at an acute angle thereto. Such acute angle preferably is in the range of about 30 to about 60 degrees to central axis 32, more preferably in the range of about 40 to about 50 degrees.

Although hood 10 is a desirable structural feature to help guide the gas flow and control the resultant spray pattern, those skilled in the art will understand that it is not an essential feature and that gas inlet 12, or other suitable nozzle, can discharge gas freely pass lumen 18 dispersing sealant drops 28 into a desired spray.

If desired, a switching control may be provided for gas stream 26 and, in a preferred embodiment, this control is integrated with suction control means for the applicator whereby gas flow is terminated when suction is applied, in a single manipulation. Alternatively, the gas stream can be controlled in a coordinated manner with the dispensing actuator such that gas flows only when liquid sealant is being driven out of the application lumen 18.

Gas flow 26 can be provided by any suitable source, for example, by a building compressed air supply, by gasses commonly available in the operating room, by a more or less portable tank or cylinder of compressed gas, or in a particularly desirable embodiment for field applications, by a small cylinder mounted on or in the spray applicator. The gas flow is preferably clean, filtered, and sterile and may comprise air, nitrogen, carbon dioxide or other inert gas, or may comprise a special-purpose gas stream which is for example, oxygen-enriched air, or carries medications or conditioners not readily incorporated in the sealant flow. If desired, the gas flow could also provide temperature-conditioning, or humidity control, or both.

The shape of mouth 14 of spray hood 10 will usually determine the shape of a spray pattern deposited on a plane surface disposed parallel to mouth 14, or generally parallel to mouth 14, if mouth 14 is non-planar. Accordingly, the shape of mouth 14 is selected to provide a desired spray pattern. As shown, spray hood 10 preferably has a varying circular cross-section perpendicularly to the paper referring to Figure 1, and is thus selected to be generally symmetrical about central axis 32 (opening 16 and the presence of lumen 18 being ignored for the purposes of this description). The inner walls of spray hood 10 are approximately parabolic, which is believed to be a helpful shape for projecting a suitably focused spray pattern. Clearly, the angle subtended by spray pattern 30 at its source, droplet 28 or applicator 19, is determined by a number of factors, including the particular dimensions of spray hood 10 and also the velocity of gas stream 26. Accordingly, the invention provides, as an optional feature, a regulator for the gas stream 26 enabling an operator to increase or decrease the width of spray pattern 30 by increasing or decreasing, respectively, the gas flow rate.

The approximately circular pattern of spray generated by the spray hood 10 depicted in Figures 1 and 2 is a useful, general-purpose shape. Figures 3 and 4 depict alternative configurations of spray hood 10 wherein mouth 14 has an elongated elliptical (Figure 3) or rectangular (Figure 4) shape which is useful for generating a wide swathe of spray 30. Other useful shapes will be apparent to those skilled in the art.

In the embodiment of Figure 5 a multiplicity of gas nozzles is arranged around the lumen 18. Nozzles 12 all have jets (not shown) such that they each emit a divergent gas stream, typically conical which gas streams converge on the axis 32 of the applicator where they impinge upon droplets or a stream of mixed sealant: 21 dispensed from lumen 18 dispersing the droplets into spray pattern that 30. Gas nozzles 12 can be selected in combination with suitable pressures to provide an air curtain 34 around spray pattern 30 to contain the spray and define the pattern.

The number of gas nozzles 12 may range from three to twelve or even twenty, or more, and optionally they may be driven by a fluidics motor 36 enabling the nozzles to be individually switched in sequence at high-speed to provide a desired spray pattern, or to compensate for the orientation of the applicator.

As referenced above, the sealant is preferably a tissue sealant or adhesive, and comprises two components or agents one agent of which contains fibrinogen and the other agent of which contains, or can generate, thrombin. The fibrinogen and thrombin agents can each or both be derived from pooled sera, a single donor or other human source, or may be autologous, according to regulatory requirements and patient needs or preferences. Additionally the thrombin agent may be derived from animal sources, bovine thrombin being commercially available and ovine and porcine being possible alternative sources. However, human sources are preferred with single donor and autologous sources being most preferred.

One method of preparing suitable tissue sealant agents from blood plasma is described in Epstein United States patents numbers 5,226,877, 5,405,607 and 5,648,265. Another method is described in Sierra United States provisional patent application No. 60/077,619. Other methods are known to those skilled in the art.

Although reference has been made to two tissue sealant agents, it will be understood that three or more agents could be employed to provide the mixed sealant 21, and other agents such as therapeutic, indicator, conditioner, or other agents can be added to or incorporated in the mixed sealant stream.

While the invention has been described in the context of generating a spray of sealant by dispensing a sealant mixture into a pressurized gaseous stream, the sealant agents may also be separately dispensed into the pressurized gaseous screen, employing known dispensing devices. It may be expected that dispersion and transport of the two or more liquid agents in the gaseous stream will provide a useful degree of mixing which may provide beneficial results at the work surface or may simply be a more convenient means of spray dispensing not requiring a dedicated spray applicator with dual spray heads.

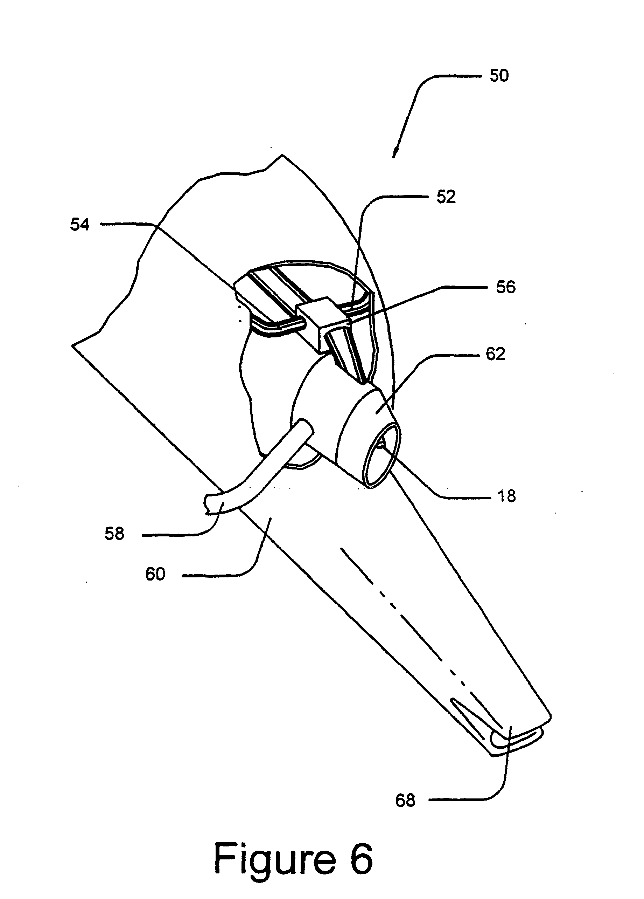

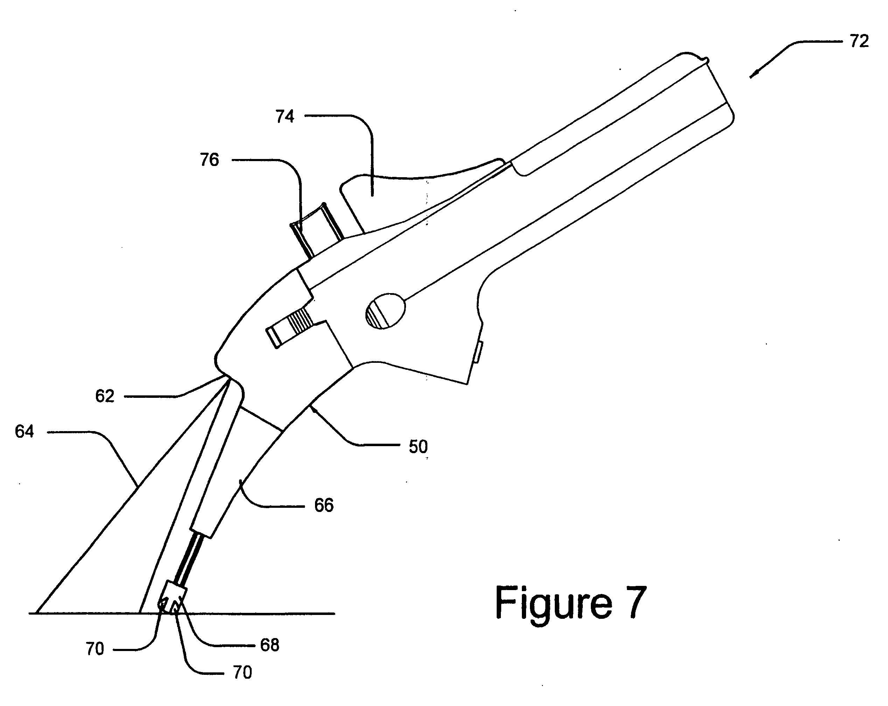

Referring to Figure 6, a spray applicator according to invention can be embodied in a trunk-like head or tip 50 intended to be remotely attached to an applicator body, such as shown in the above-referenced application number PCT/US99/04830, and also in Figure 7, (described below) which applicator body serves to dispense two streams of fluid sealant agents to conduits 52 and 54, leading to mixing head 56, where the agents are mixed and dispensed as a mixture into lumen 18. Gas is supplied by a line 58, which could be concealed within the device, from a suitable source, which could be a small gas tank carried in the applicator body, to a gas nozzle 60. Gas nozzle 60 is positioned to disperse sealant from a lumen 18 into a spray 62, discharging the spray from spray tip 50 through a suitably dimensioned spray port 62. Though not shown, it is preferred to provide a suitably shaped, cup-like hood within spray tip 58 whose mouth coincides with spray port 62 and which fits closely around nozzle 60 and lumen 18.

Spray tip 50 has a protruding nose 66 to serve as a spacer to maintain a desired distance from the work surface. Nose 66 is optionally fitted with an extensible portion 68 to vary that distance. Suction can be applied through an aperture or apertures 70 in nose 66 or extensible portion 68, if present, to prepare the work surface.

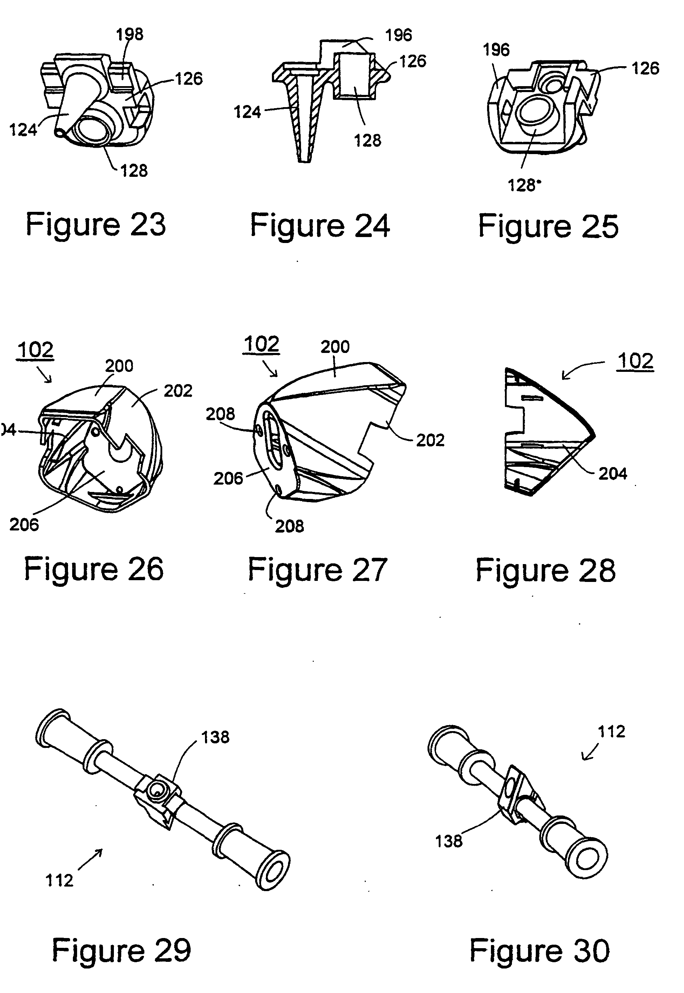

Figure 7 shows how a spray applicator such as described in connection with Figure 6 can be embodied in a dual agent sealant applicator with the outward appearance of that shown in Figure 27 of application number PCT/US99/04830. Spray tip 50 is shown assembled with an applicator body 72 whose internal mechanism is described in that application and includes two syringe-like sealant agent reservoirs (not showed here) from which a sealant is dispensed by repeatedly pressing actuator trigger 74. Preferably, the sealant agent reservoirs are refillable.

Suction and retrograde clearing are effected by control button 76, while suction is supplied from a suitable external source via suction adapter 78. Control button 76 preferably actuates a suction valve, not shown, which balances applied suction and venting to atmosphere, permitting accurate control of suction applied to the work surface. Control button 76 preferably has an at-rest position where no suction is applied by applicator body 72, a suction-applying position where controlled suction can be applied to a work surface through an attached tip assembly and a suction clearing position where suction can be applied to a mixed sealant pathway in an attached tip assembly.

Applicator body 72 is designed to receive one or another of a range of interchangeable tips providing a variety of functions. Such tips may include a droplet tip for dispensing drops or a stream of sealant, the sealant being mixed in the tip from the two sealant agents stored in reservoirs in applicator body 72. Such tips are described in various ones of the copending applications referenced herein. Alternatively the applicator tip may comprise a spray tip capable of dispensing a controlled spray of sealant, the sealant being mixed in the tip from the two sealant agents stored in reservoirs in applicator body 72. A further alternative is for the applicator tip to comprise a filler assembly from which supplies of the sealant agents may be re-filted into the reservoirs in applicator body 72.

The present invention also provides, in preferred embodiments, spray tip assemblies suitable for use with applicator body 72 and it extends to a spray applicator comprising a spray tip assembly attached to such an applicator body.

Atomizer, or impeller gas may, if desired, be provided from an on-board cylinder 82 and be controlled by actuator trigger 74, control button 76 or a separate control member (not shown). Extensible portion 68 of nose 66 acts as a feeler and is drawn, or advanced, along a work surface 80, maintaining a desired spacing while suction through ports 78 prepares the work surface and spray 64 is applied, as desired.
Alternatively, suction preparation and spray application may be alternated.

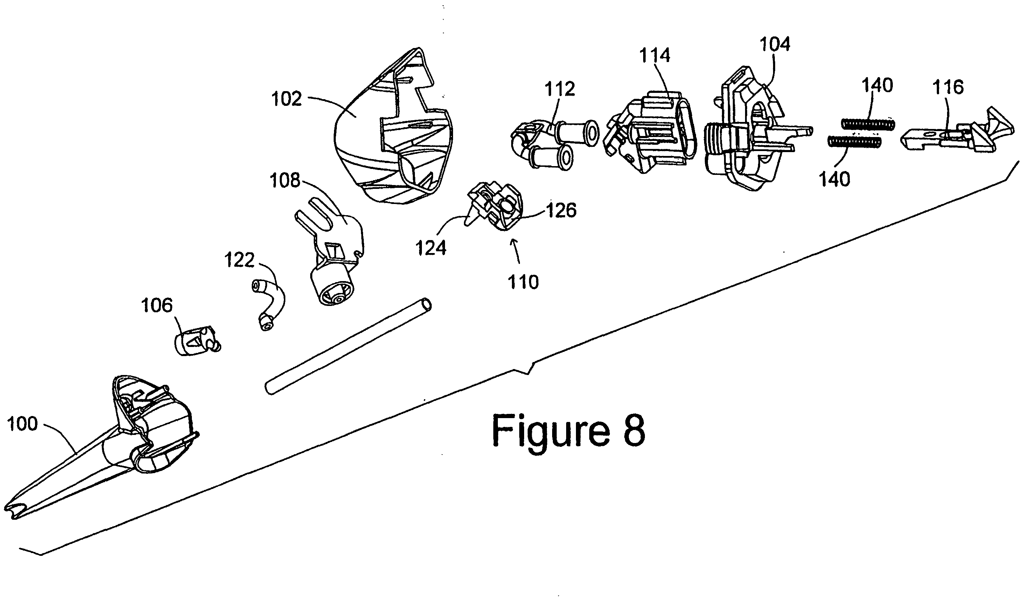

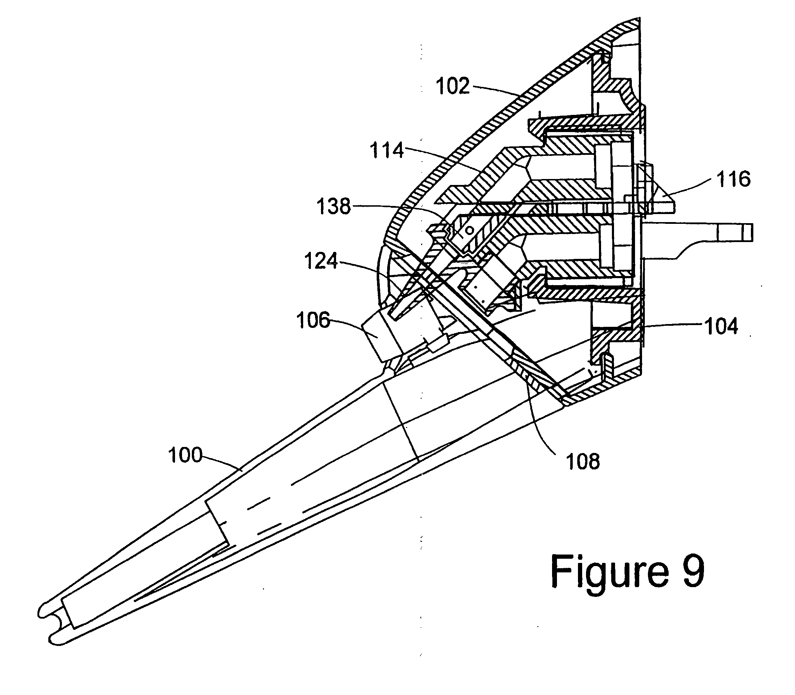

Referring now to Figures 8 and 9, the spray tip assembly shown is intended to provide, when attached to an applicator body such as applicator body 72, an air-driven spray applicator for dispensing a spray pattern of, for example, a mixture of two or more sealant agents which, in a preferred embodiment, are polymer and activator agents of a fibrin sealant for use in surgical procedures. The spray tip assembly comprises a suction nose 100, a cover 102 and an end plate 104 which together comprise the principal external agents of the assembly. Suction nose 100 and cover 102 which together provide the external surfaces of the spray tip assembly are configured and finished to provide an esthetic and ergonomic external shape and feel to the applicator instrument when the spray tip assembly is assembled with an applicator body such as applicator body 72. Other useful functional characteristics are also provided, as will be explained in, or apparent from, the following description. End plate 104 is engageable with cooperative structure on the applicator body to permit the spray tip assembly to be securely and releasably attached to the applicator body to receive sealant agents and controlled suction therefrom.

Supported within the assembly are a spray nozzle 106, a spray nose plate 108, an application tip 110, a manifold 112, a vacuum tube assembly 114 and a shuttle valve 116 which latter agent projects proximally through end plate 114. Spray nose plate 108 is clamped in place between suction nose 100 and cover 102 and comprises a generally diamond-shaped flat yoke 118 and a compressed air adapter 120. Yoke 118 fits around lumen 124 and a suction tube and locates spray nose plate 108 within the spray assembly. Air adapter 120 provides an external fitting for the connection of a compressed air line (not shown). An elbow-shaped connector tube 122, formed, for example, of silicone rubber, carries pressurized air from adapter 120 to spray nozzle 106.

Application tip 110 comprises a tapered lumen 124 which mates with manifold 112 to receive mixed sealant therefrom and discharge the sealant into nozzle 106.

U-shaped, flexible manifold 112 brings the sealant agents from applicator 72, mixes them and discharges the mixed sealant into lumen 124. Application tip 110 has a flanged base plate 126 provided with a suction connector 128, which base plate 126 matingly engages with the distal end of vacuum tube assembly 114. A suction tube, which may be formed, for example, of polyethylene, supplies suction from port 128, in anchor plate 126 of application tip 110, to suction nose 100.

A vacuum tube assembly 114 matingly engages with end plate 104 which end plate 104 supports and locates vacuum tube assembly 114. Vacuum tube assembly 114 is in its turn received between the arms of U-shaped manifold 112 and supports manifold 112. Vacuum tube assembly 114 has two internal suction passages arranged one above the other, namely, an upper suction clearing passage 132 and a lower suction applying passage 134. Both suction passages 132 and 134 are accessible externally of the spray tip assembly through a central opening 136 in end plate 104 where they can mate with suitably configured suction ports or connectors on applicator body 72 and be supplied with suction from a suction source in a manner controllable by the user, employing manual suction control valve 76.

Suction clearing passage 132 communicates with a mixing chamber 138 in manifold 114 to and thence with lumen 124 which is aligned to receive sealant from mixing chamber 138. Suction applying passage 134 communicates with the suction tube through suction port 128 in anchor plate 126, to be applied to a work surface through suction nose 100.

Shuttle valve 116, moving through vacuum tube assembly 114, acts on flexible manifold 112 to close off the supply of sealant agents from applicator body 72 and to communicate mixing chamber 138 with suction clearing passage 132, to permit retrograde clearing of lumen 124 and mixing chamber 138. Springs 140 bias shuttle valve 116 into a sealant applying position where manifold 112 is open to convey sealant agents to mixing chamber 138. In this position, shuttle valve 116 closes the top of mixing chamber 138, preventing application of suction thereto.

The agents of the suction and sealant delivery system comprised by manifold 112, vacuum tube assembly 114, end plate one of all, shuttle valve 116 and springs 140, as shown, are structurally similar to the corresponding agents of a droplet applicator tip as described in applicant's copending application No. PCT/US99/04830, where the agent here described as vacuum tube assembly 114 is there described as lefthand and righthand clamshells 108. Cover 102 is designed to enclose and support the functional agents. The downstream agents, suction nose 100 spray nozzle 106 spray nose plate 108 and application tip 110, as well as connector tube 122 and the suction tube are particularly adapted for spray discharge of sealant, as opposed to delivering a sealant stream or droplets externally of the applicator tip.

In an alternative embodiment of the invention, not shown, these downstream agents could be fabricated as a separate unit to provide a readily removed functional unit adapting the sealant applicator for spraying. Similarly, the droplet applicator of the parent application could be correspondingly constructed to have those parts downstream of mixing chamber 138 fabricated as a removable unit, thus providing relatively small, interchangeable units. The agents upstream of application tip 110 could be incorporated into applicator body 72, but more preferably would be fabricated as a separate removable unit, to permit refilling of applicator body 72 and access to internal agents for service and replacement.
Suitably modified constructions of cover 102, providing enclosure and support functions, will be apparent to those skilled in the art.

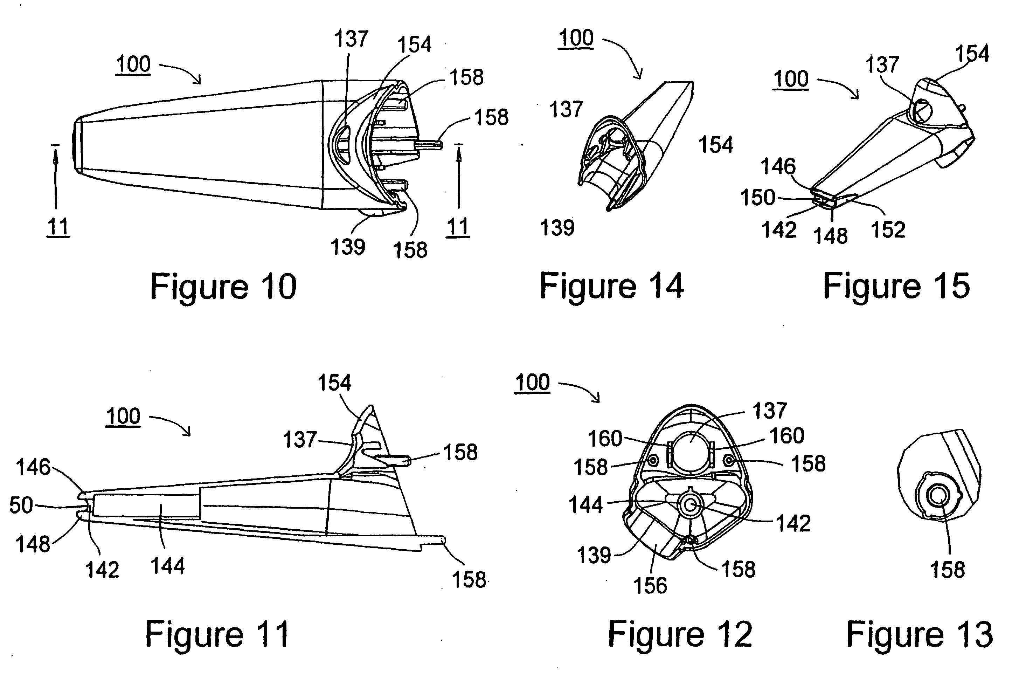

Referring now to Figures 10-15, suction nose 100 is hollow with an elongated configuration designed for secure attachment to cover 102 at its proximal end (the righthand end as viewed in Figures 10 and 11) and to apply suction to a work surface at its distal end (the left-hand end as viewed in Figures 10 and 11). At its proximal end, suction nose 100 also has a sprayer opening 137 to receive spray nozzle 106 and a cutout 139 to accommodate a compressed air adapter element of spray nose plate 108.

At its distal end, suction nose 100 has a suction applying aperture 142 which communicates internally with a tubular socket 144 into which socket the suction tube is received. Externally, at the distal, suction-applying end, suction nose 100 is formed with elongated, parallel upper and lower lips 146 and 148 respectively above and below aperture 14 to which is approximately centrally located with respect to the lips 146-148. It will be appreciated that instead of a single centrally located orifice, suction aperture 142 may comprise multiple openings, or an elongated opening, located between upper lip 146 and lower lip 148.

Between lips 146 and 148, suction nose 100 comprises a channel 150 the effect of which is to recess suction aperture 142 behind lips 146-148 to deter grabbing of soft or loose work surface elements, for example tissue exposed during surgery, which might occur were suction aperture 142 to become blocked. Side vents 152 (Figure 15) facilitate passage of work surface liquids to suction aperture 142 and also help prevent grabbing. Preferably, the distal end agents of spray nose 100 are smoothly contoured and finished so as to provide a high-quality probe suitable for engagement with and movement across surgical tissues without sharp edges, corners or molding seams that might damage, or displace, delicate tissues, or harbor infection. While lips 146 and 148 are shown in Figures 10, 11 and 15 as being substantially straight, they can, if desired, be smoothly curved, particularly toward their ends, where they merge into the body of suction nose 100.

As its proximal end, and on its upper surface, spray nose 100 has an upstanding cowling 154 which is formed centrally with sprayer opening 137 and is shaped to merge smoothly into cover 102. A small semicircular wall 156 projects downwardly from the lower surface of spray nose 100 and defines cutout 139 which accommodates the compressed air adapter. Three locking posts 158, having transverse structures, for example, as shown in the enlarged view of Figure 13 are received into cooperative openings in cover 102 enabling spray nose 100 and cover 102 to be securely assembled together. Abutments 160 can optionally be provided to guide and support spray nozzle 106 in sprayer opening 137.

The length of spray nose 100 is chosen to provide a desired spacing of spray nozzle 106 from the work surface, whereby spray nose 100 functions as a spacer as well as a suction applicator. In addition, the spacer function of spray nose 100 supplements patient safety by keeping the air stream from spray nozzle 106 at a safe distance from sensitive patient tissue which could be damaged by undue air pressure.

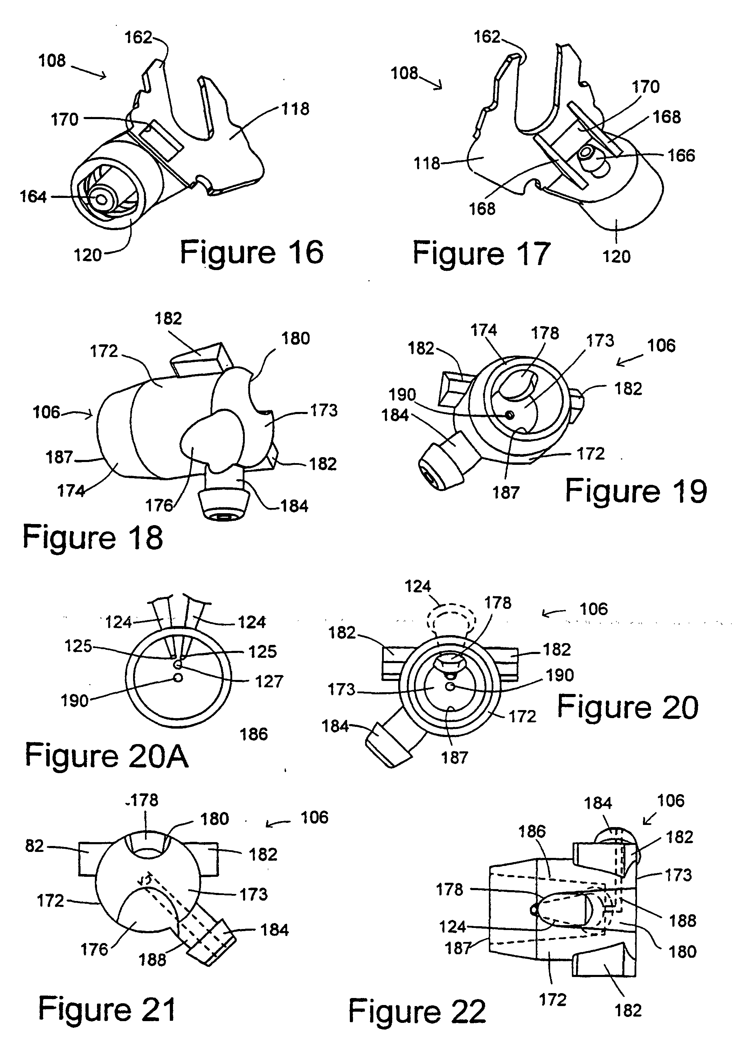

Referring now to Figures 16 and 17. yoke 118 of spray nose plate 108 is configured to be clamped snugly within suction nose 100 by cover 102 and has a U-shaped cutout 162 to accommodate sealant-dispensing lumen 124 and suction tube 130, both of which pass through yoke 118. Air adapter 120 is generally cup-shaped and projects externally through cutout 139 in suction nose 100, where it is provided with a standard fitting 164 to receive a resilient air hose. Intemally of the spray tip assembly, air adaptor 120 comprises a nipple 166 to receive connector tube 122. Small buttresses 168 between yoke 118 and adapter 120 provide support and rigidity and a rectangular opening 170 in yoke 118 facilitates moldability.

Spray nose plate 108 is configured, particularly with regard to the shape of yoke 118, to have a periphery that fits closely within the proximal end of spray nose 100, providing a closing plate to spray nose 100. Spray nose plate 108 engages with the internal structure of spray nose 100, especially within cowling 154 and cutout 139, so as to fit securely and provide a solid and sturdy bearing surface for engagement with cover 102.

Referring now to Figures 18-22, spray nozzle 106 comprises a generally cylindrical spray body 172 which has a proximal end wall 173 and an externally tapering distal tip 174. The underside of spray body 172 has a shallow, curved recess 176 to accommodate the suction tube (Figure 18). An elongated opening 178 is formed in the top wall of spray body 172 and a groove 180 is formed in end wall 173 to receive and locate lumen 124 (Figure 19). Prism-shaped wings 182 extend either side of opening 178 and engage tightly with cooperative structure within cowling 154 on suction nose 100 to lock spray nozile 106 in place. A flanged connector 184 projects radially from the lower side of spray body 172 to receive the other end of connected tube 122 and supply compressed air to spray nozzle 106 from air adapter 120.

Internally, spray body 172 has a spray chamber 186 (Figure 22) which defines the spray volume where the spray originates and which opens into a mouth 187 at distal tip 174. Spray chamber 186 tapers in the proximal direction to encourage formation of a divergent or conical spray. The degree of tapering is related to the desired spray pattern and the length of suction nose 100 which controls the spacing of spray of nozzle 106 from the work surface. The cross-sectional chamber spray pattern is also largely determined by the cross-sectional shape of spray chamber 186, especially at the mouth 192 of spray chamber 186, which in the embodiment shown is circular. An air passage 188 extends through connector 184 and end wall 173 to open in an orifice 190 located in the center of end wall 173, within spray chamber 186.
Lumen 124 is shown in broken lines in Figures 20 and 22 terminating just above orifice 190.

If desired, spray body 172 can be of variable configuration (not shown), for example it may be telescopic, to enable a user to vary the cone angle of the spray pattern generated. Thus, a wider spray, with a larger cone angle, may be generated by enlarging mouth 175 of spray body 172 or by shortening spray body 172 to bring mouth 175 closer to end wall 173. A control such as a knob, a dial or a lever can be provided to enable a user to effect such variation.

Also, spray chamber 186 may have a shape other than the cylindrical shape shown. For example, rather than a circular cross-section, spray chamber 186 may have an elliptical or other cross-sectional shape as described hereinabove or otherwise as will be apparent to those skilled in the art. Furthermore, while only one centrally disposed nozzle or orifice 190 is shown, if desired multiple orifices could be used, provided that a single gas stream emerges from spray chamber 186.

Referring to the alternative embodiment shown in Figure 20, two lumens 124 separately deliver the sealant agents to spray chamber 186 to be discharged as a single stream of spray entrained in the gas flow emerging from gas orifice 190. Preferably, lumens 124 have tips 125 which are sufficiently spaced apart to prevent contamination of one lumen with a sealant agent from the other lumen, which could cause clogging, yet are sufficiently close together to avoid generation of one or more separate zones of a single sealant agent. More preferably, lumens 124 are disposed somewhat as shown in Figure 20A with both tips 125 positioned a little above orifice 190 and sufficiently close together that liquid drops or streams from the two lumens merge and coalesce into a mixed droplet 127, as they descend, assuming the spray applicator to be held in an upright position with lumens 124 having the orientation shown. This being the case, the degree of merging or coalescing of the two liquids can be expected to depend upon the velocity of the gas stream emerging from orifice 190.

The invention is not limited to any particular size of apparatus or agent. However, one embodiment of spray nozzle or spray hood 10 suitable for use with a surgical sealant applicator having a body length (without spray tip assembly) of about 15 cm (6 in.) has a diameter at mouth 14 of about 5 mm (0.2 in.), with other dimensions being approximately in proportion.

Referring to Figures 23-25, lumen 124 depends downwardly from base plate 126 of application tip 110. Upwardly, lumen 124 communicates with mixing chamber 138 in manifold 112. Suction connector 128 projects downwardly from the underside of base plate 126 to receive suction tube 130 and projects upwardly from base plate 126 to connect with suction applying passage 134 of vacuum tube assembly 114. Arches 196 extend upwardly on either side of suction connector 128 for snap engagement with cooperative structure on vacuum tube assembly 114. Distally extending hooks (not referenced) on vacuum tube assembly 114 snap under recesses 198 on either side of lumen 124. Thus, application tip 110 is securely assembled with vacuum tube assembly 114 to receive sealant and suction therefrom, in a manner more fully described in connection with the droplet applicator shown in copending application PCT/US99/04830.

Cover 102 is designed to receive application tip 110, manifold 112 and vacuum tube assembly 114 assembled together as a unit, which unit may, optionally, be mounted on plate 104 for assembly with cover 102. Externally, cover 102 is shaped to be smoothly and aesthetically contoured and finished, to be readily gripped and manipulated between the thumb and forefingers of most users and to enclose and support the just-described functional agents of the spray tip assembly. Helpful features in this respect are an upper flat 200 of limited lateral extent and adjoining side faces 202 angled to flat 200.

Internally, cover 102 has suitable support structure such as rib 204 to engage with and to support and locate vacuum tube assembly 114 and the agents assembled therewith. A flat distal end wall 206 mates cleanly with spray nose plate 108 and has small peripheral openings 208 receives posts 158 projecting of proximally from suction nose 100 permitting cover 102 to be securely attached to suction nose 100 by suitable fastening means, clamping spray nose plate 108 in place and locking spray nozzle 106 into position in cowling 154. Attachment of cover 102 to suction nose 100 is conveniently effected prior to assembly of vacuum tube assembly 114, and its accompanying agents, into cover 102.

A large central opening 208 in end wall 206 provides access to spray nozzle 106 via cutout 139 in spray nose plate 108. The underside of base plate 126 rests on yoke 118 of spray nose plate 108, with lumen 124 and suction connector 128, with suction tube 130 attached, extending through opening 208 and cutout 139. Lumen 124 extends downwardly into spray chamber 186 of spray nozzle 106 to deliver sealant thereto, while the suction tube applies suction to suction aperture 142 at the distal end of suction nose 100.

A compressed air stream emerging from orifice 190 entrains sealant delivered from lumen 124 and generates a mixed sealant spray which emerges from spray nozzle 106 with a pattern determined by the internal geometry of spray nozzle 106. If desired, a compressed air control (not shown) can be coupled with actuation of the sealant applicator to provide air output during sealant delivery, and stopping when sealant delivery is terminated.

Figures 29-34 show more details of certain components of the spray tip assembly, namely manifold 112, vacuum tube assembly 114 and shuttle valve 116. These components have generally similar structure and function to the corresponding components manifold 102, clamshell 108 and shuttle valve 54 , described in detail in my application number PCT/US99/04830, but differ from the corresponding components with regard to certain details, as may be seen from the respective figures.

Thus, for example, referring to Figures 29-30, mixing chamber 138 of manifold 112 has a generally rectangular external shape, in section, and is configured to be an accurate mating fit with base plate 126 of application tip 110 (see Figure 25).

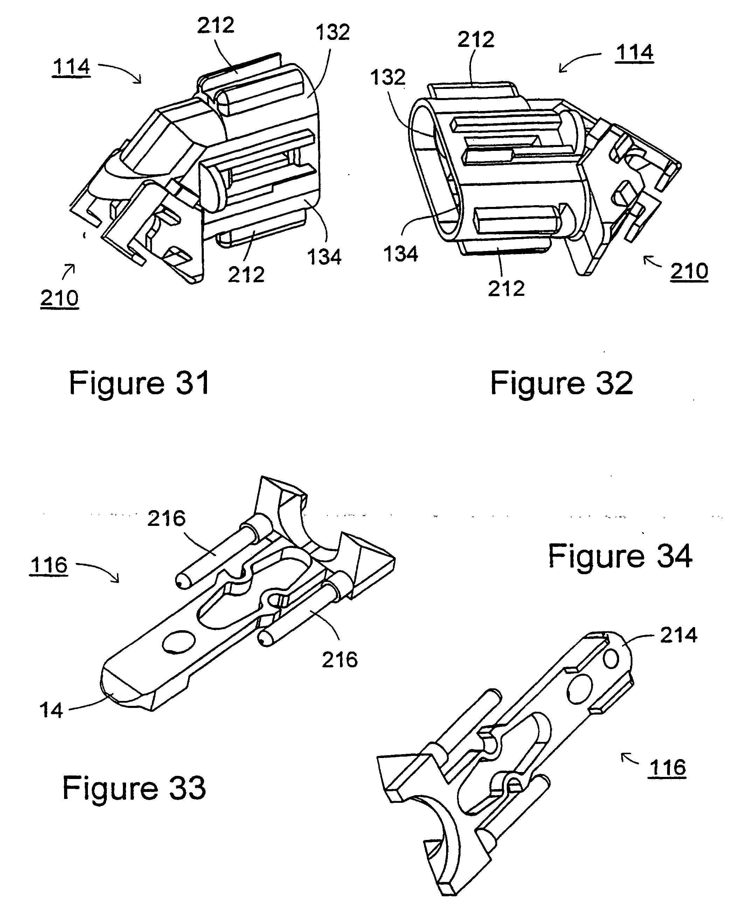

Referring to Figures 31-32, vacuum tube assembly 114 is configured to be capable of being molded in one-piece rather than in two halves Referring to Figures 31-32, vacuum to assembly 114 is configured to be capable of the molded in one-piece rather than in two halves, has modified manifold-engaging structure indicated generally at 210, to facilitate assembly and location of manifold 112, and has a number of longitudinal ribs, such as 212, which strengthen and locate manifold 112 with end plate 104, providing a stable construction.

Referring to Figures 33-34, shuttle valve 116 has a proximal nose portion 214 to function as a shock-absorbing bumper and provide an effective end stop. Side rods 216 carry compression springs 140 which resiliently bias shuttle valve 116 in the distal direction. Cam members 218, which are engaged by cooperative structure in the applicator body to advance shuttle valve 116 distally, are connected by a cradle 220 for structural rigidity.

Other functional and structural details of these and other components of the spray the assembly will be apparent from the drawings.

The various components of the spray tip assembly shown in Figures 8-34 are suitable for molding from synthetic plastic materials, in most cases in the one piece shown for the given component. Some examples of suitable synthetic plastic materials are: medical grade ABS (acrylonitrile butadiene styrene), for example as supplied by Bayer AG under the trademark LUSTRAN, which may be used for suction nose 100, cover 102, spray nozzle 106 and spray nose plate 108; medical grade polycarbonate, for example as supplied by Bayer AG under the trademark MAKROLON, which may be used for end plate 104, vacuum tube assembly 114 and shuttle valve 116; and medical grade silicone, for example as supplied by Dow under the trademark SILASTIC or by Bayer AG under the trademark BAYSILONE, which may be used for manifold 112 and preferably has a durometer of 40-60 (Shore A). Other possible materials and modes of manufacture of the components will be apparent to those skilled in the art.

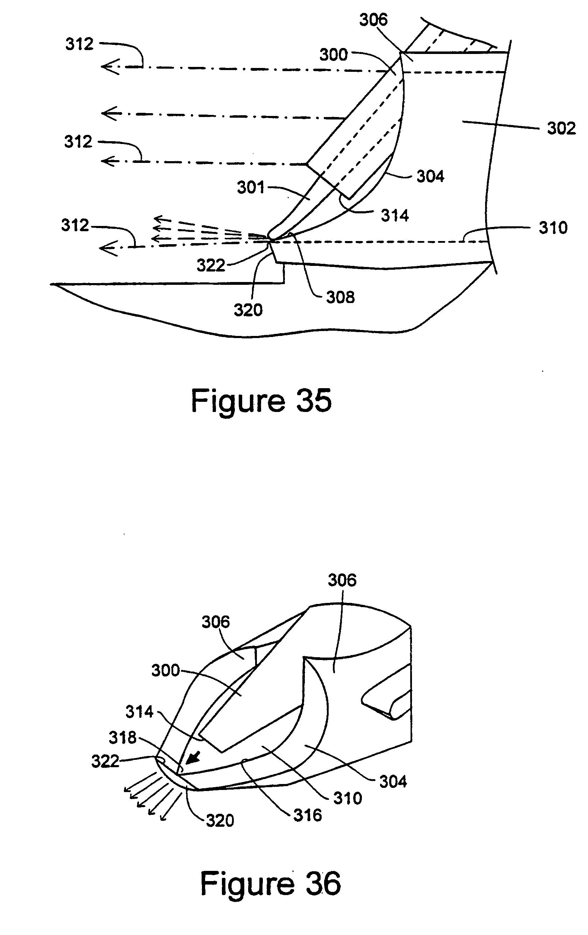

Pursuant to a further aspect of the invention, illustrated by way of example in Figures 35-36, a gas-driven spray device is provided which comprises a liquid-dispensing aperture providing a source of liquid to be sprayed, a shearing plate and a gas nozzle to provide a stream of carrier gas wherein the liquid can be discharged from the liquid-dispensing aperture to impact the shearing plate and the gas flow is disposed in relation to the shearing plate to carry liquid droplets away from the shearing plate in a spray. Preferably the liquid or other material to be dispensed is discharged under pressure, from a lumen, toward and onto the shearing plate across a gap through which gas air stream flows. Preferably also, the lumen is oriented so that the direction of fluid discharge has a significant component in the direction of gas flow.

Referring now to the gas-driven shear-spray device shown in Figures 35-36, a dispensing lumen 300 is disposed to discharge a liquid sealant 301, or other sprayable liquid, as droplets or continuous stream, downwardly across a gas nozzle 302. It will be understood that lumen 300 could be lumen 18 or lumen 124, as described hereinabove, from which a stream of mixed sealant agents can be dispensed by a hand-held applicator and that gas nozzle 302 is an alternative to spray nozzle 106 shown hereinabove.

Gas nozzle 302 has a cut away mouth 304 defined by set back shoulders 306, which embrace and support lumen 300, and terminates proximally in a shear plate 308. Gas nozzle 302 has a hollow interior chamber 310 communicating with a gas source which can generate a gas stream 312 flowing past the tip 314 of lumen 300. A proximally tapering groove 316 defined in the floor of chamber 308 leads to shear plate 306. Shear plate 308 comprises an impact surface 318, a proximal face 320 at a relatively large acute angle thereto, or approximately perpendicular thereto, and a sharp shear edge 322 defined where impact surface 318 and face 320 meet. Lumen 300 is preferably oriented to direct emerging sealant 301 so that it strikes shear edge 322 directly, or alternatively strikes shear plate 308. In use, the precise point of impact may vary, depending upon the velocity of discharge of sealant 301 and the velocity of transversely moving gas stream 312. Alternatively to dispensing mixed sealant agents from lumen 300, a single liquid sealant, or sealant agent, could be dispensed, or multiple lumens 300, dispensing multiple sealant agents or other liquid agents, could be directed at shear plate 308.

Sealant 301 discharged from lumen 300 impacts on surface 318 and is driven across shear edge 322, by the momentum of discharge, and by gas stream 312. The combined effect of shear edge 322 and gas stream 312 breaks the sealant flow into droplets, which are dispersed into gas stream 312, as indicated schematically by arrows 324, generating the desired spray. Shear plate 308 stops the flow in the downward direction, thins the sealant and positions it so that gas stream 312 can force the thinned layer of sealant or other liquid to shear off shear plate 308 into a fine spray.

Shear plate 308 aids in the atomization of sealant agents and enables an effective spray to be generated at relatively high sealant discharge velocities at which the sealant stream might otherwise pass through the gas stream, avoiding atomization, were shear plate 308 not present.

The cut away configuration of the device provided by set back shoulders 306 is valuable in providing a user with full visibility of the tip 31 of lumen 300, and also for providing access thereto, to remove clogs, if necessary.

While lumen tip 314 is shown disposed within gas stream 312, it could be disposed outside the gas stream, and discharge liquid into the gas stream. Similarly shear plate 308, or the output from shear plate 308, could be disposed outside the gas stream, and the gas stream could be disposed to entrain the fluid droplets exiting from shear plate 308.

Such a gas-driven shear-spray device is particularly useful for atomizing macromolecular fluids, for example sealants, including fibrin sealants whose dynamic rheology impedes atomization. Significant forces at the shearing plate acting positively on the cohesive large molecules of the sealant help divide the liquid into fine particles for dispersion. Other liquids that may be beneficially sprayed employing such a gas-driven shear-spray device, for example paints, coatings and the like, will be apparent to those skilled in the art.

While illustrative embodiments of the invention have been described above, it is, of course, understood that various modifications will be apparent to those of ordinary skill in the art.

## Claims

1. A spray applicator for spraying a liquid sealant comprising at least two sealant agents capable of coagulating when mixed together, the applicator comprising:
a) a spray volume;
b) a sealant delivery pathway (18, 124) extending from individual sources of the respective sealant agents to the spray volume and adapted to mix the at least two sealant agents to provide a mixed sealant (21) and to deliver the mixed sealant to the spray volume;
c) a pressurized gas inlet (12, 302) adapted to admit a gas stream (26) to the spray volume;
wherein the sealant delivery pathway (18, 124) is adapted to deliver the mixed sealant into the gas stream (26) such that a spray containing the mixed sealant is generated in the spray volume; and
d) a clearing device adapted to clear clogged sealant material from the dispensing pathway (18, 124) by applying suction.

2. A spray applicator according to claim 1, further comprising a spray chamber defining the spray volume, the spray chamber having a mouth (14, 187), having the pressurized gas inlet (12, 302) disposed remotely from the mouth (14, 187) and having a sealant agent delivery region disposed between the mouth (14, 187) and the gas source whereby the gas stream (26) passes through the spray volume from the gas source to the mouth (14, 187) and can receive and entrain the sealant agents in the delivery region.

3. A spray applicator according to claim 2, wherein the sealant dispensing pathway (18, 124) comprises a lumen having a sealant dispensing aperture (19) disposed in the spray chamber in a location where the dispensing aperture is surrounded by the gas stream (26).

4. A spray applicator according to claim 3, wherein the sealant dispensing aperture (19) is positioned upstream of the mouth (14, 187) of the spray chamber, does not project through the mouth (14, 187) and is disposed at an acute angle to the direction of flow of the gas stream (26) so that sealant emerging from the dispensing aperture is drawn away from the lumen by the gas flow.

5. A spray applicator according to claim 1, wherein the pressurized gas inlet (12, 302) is connectable to a source of pressurized gas being a building compressed air supply, a surgical operating room gas supply or a portable container of compressed gas.

6. A spray applicator according to claim 5, wherein the pressurized gas is clean, filtered, sterile inert gas, air, nitrogen, carbon dioxide, oxygen-enriched air, or a gas carrying a medication or work surface conditioner.

7. A spray applicator according to claim 5 or 6, further comprising a spacer element (66, 100) extending in the direction of spraying to maintain a desired distance between the spray volume and a work surface.

8. A spray applicator according to claim 1, 2, 3 or 4, further comprising a suction pathway connectable with a suction source and having a suction aperture for applying suction to a work surface to be sprayed.

9. A spray applicator according to claim 7, further comprising a suction pathway connectable with a suction source and having a suction aperture for applying suction to the work surface to be sprayed wherein the suction aperture is located in the spacer element (66, 100).

10. A spray applicator according to claim 1, 2, 3 or 4, wherein the at least two sealant agents are delivered to the spray volume by manual operation of a manual actuator.

11. A spray applicator according to claim 10, further comprising a lumen extending from the mixing volume to the spray volume, the lumen having a dispensing aperture disposed in the mixing volume for delivering the mixed sealant (21) to the spray volume.

12. A spray applicator according to claim 11, wherein the lumen projects into the spray volume to be swept by the gas stream (26) from the gas inlet (12, 302).

13. A spray applicator according to claim 1, wherein the clearing device is connectable with the sealant dispensing pathway (18, 124) to apply retrograde suction to the mixing volume.

14. A spray applicator according to claim 1, 2, 3 or 4, wherein the sealant is a biological sealant suitable for surgical use.

15. A spray applicator according to claim 14, wherein the sealant is a fibrin sealant comprising a fibrinogen agent and a fibrinogen activator agent, said fibrinogen agent optionally being thrombin.

16. A spray applicator according to claim 14, further comprising at least two reservoirs serving as sources for the at least two sealant agents.

17. A spray applicator according to claim 16, further comprising an exterior housing and having a size, configuration and arrangement suitable for the applicator to be held and operated in one hand.

18. A spray applicator according to claim 2, 6, 7 or 9, wherein the spray chamber has a shape diverging in the direction of flow of the gas stream (26).

19. A spray applicator according to claim 18, wherein the spray chamber is shaped to generate a spray occupying a volume which diverges in the direction of travel of the spray.

20. A spray applicator according to claim 2, 6, 7 or 9, wherein the sealant agents comprise a coagulating sealant mixture in the sealant delivery pathway before delivery to the gas stream (26).

21. A spray applicator according to claim 2, 6, 7 or 9, wherein the sealant agents are moved along the delivery pathway under pressure and released into the gas stream.

22. A spray applicator according to claim 21, wherein the applicator comprises reservoirs for the sealant agents, a manual actuator and a drive mechanism to apply manual force and drive the sealant agents out of the reservoirs under pressure.

23. A spray applicator according to claim 1, further comprising an applicator body and a spray tip assembly removably attached to the applicator body, wherein the applicator body has:
a) at least two reservoirs respectively for storing the at least two sealant agents;
b) at least two conduits for separately dispensing the at least two sealant agents; and
c) a manually actuatable dispensing mechanism to discharge the sealant agents from the reservoirs through the at least two conduits;
wherein the spray tip has assembly has:
d) at least two sealant agent receiving ports connectable with the at least two conduits;
e) a mixing chamber to receive and mix the at least two sealant agents from the at least two sealant receiving ports to provide the mixed sealant;
f) the spray volume;
g) a delivery conduit to receive mixed sealant from the mixing volume and deliver the mixed sealant to the spray volume; and
h) the pressurized gas inlet (12, 3 02);
and wherein the delivery conduit extends into the path of the gas stream (26) to deliver the mixed sealant to the gas stream (26) and generate the spray of mixed sealant droplets.

24. A spray applicator according to claim 23, wherein the spray tip assembly comprises a spacer element (66, 100) extending in the direction of spraying to maintain a desired distance between the spray volume and a work surface.

25. A spray applicator according to claim 23, wherein the spray tip assembly comprises a suction pathway (18, 124) connectable with a suction source and having a suction aperture for applying suction to a work surface to be sprayed wherein the suction aperture is located in the spacer element (66, 100).

26. A spray applicator according to claim 23, wherein the clearing device is connectable with the mixing volume to apply retrogade suction to clear the mixing volume and the delivery conduit.

27. A spray applicator according to any of claims 23 to 26 which is adapted to be held and operated in one hand.

28. A spray tip assembly for use in the spray applicator of claim 23;
wherein the spray tip assembly comprises:
d) at least two sealant agent receiving ports connectable with at least two conduits;
e) a mixing chamber to receive and mix the at least two sealant agents from the at least two sealant receiving ports to provide the mixed sealant;
f) the spray volume;
g) a delivery conduit to receive the mixed sealant from the mixing volume and to deliver the mixed sealant to the spray volume;
h) the pressurized gas inlet (12, 302); and
i) a clearing device adapted to clear clogged sealant material from the dispensing pathway by applying suction;
wherein the delivery conduit extends into the path of the gas stream (26) to deliver the mixed sealant to the gas stream (26) to generate the spray of mixed sealant droplets.

29. A spray tip assembly according to claim 28, further comprising a spacer element (66, 100) extending in the direction of spraying to maintain a desired distance between the spray volume and a work surface.

30. A spray tip assembly according to claim 29, further comprising a suction pathway connectable with a suction source and having a suction aperture for applying suction to a work surface to be sprayed wherein the suction aperture is located in the spacer element (66, 100).

31. A spray top assembly according to any of claims 28 to 30, wherein the clearing device is connectable with the mixing volume to apply retrogade suction to clear the mixing volume and the delivery conduit.

## Patentansprüche

1. Spray-Applikator zum Versprühen eines flüssigen (Gewebe-)Klebstoffes bzw. eines flüssigen Verbandes bzw. einer flüssigen Dichtungsmasse, welcher bzw. welche wenigstens zwei Klebstoff- bzw. Verbands- bzw. Dichtungsmassen-Agenzien umfaßt, welche imstande sind, zu gerinnen bzw. zu koagulieren, wenn sie zusammengemischt werden, wobei der Applikator folgendes umfaßt:
a) ein Spray-Volumen;
b) eine Klebstoff-Zuführungsleitung (18, 124), welche sich von einzelnen Quellen des jeweiligen Klebstoff-Agens zum Spray-Volumen hinerstreckt und eingerichtet ist, die wenigstens zwei Klebstoff-Agenzien zu mischen, um einen gemischten Klebstoff (21) bereitzustellen und um den gemischten Klebstoff dem Sprayvolumen zuzuführen;
c) einen Einlaß (12, 302) für Druckgas, welcher eingerichtet ist, einen Gasstrom (26) in das Spray-Volumen einzulassen;
wobei die Klebstoff-Zuführungsleitung (18, 124) eingerichtet ist, den gemischten Klebstoff dem Gasstrom (26) zuzuführen, so daß in dem Spray-Volumen ein Spray erzeugt wird, welches den gemischten Klebstoff beinhaltet; und
d) eine Freimach-Einrichtung, welche eingerichtet ist, um verklumptes Klebstoff-Material durch die Anwendung einer Absaugung aus der Zuführungsleitung (18, 124) zu beseitigen.

2. Spray-Applikator nach Anspruch 1, der weiterhin eine Spray-Kammer umfaßt, welche das Spray-Volumen definiert, wobei die Spray-Kammer einen Eingang (14, 187), den Einlaß (12, 302) für Druckgas, welcher entfernt vom Eingang (14, 187) angeordnet ist, und einen Klebstoff-Agens-Zuführungsbereich hat, welcher zwischen dem Eingang (14, 187) und der Gasquelle angeordnet ist, wodurch der Gasstrom (26) das Spray-Volumen von der Gasquelle zum Eingang hin (14, 187) durchströmt und die Klebstoff-Agenzien im Zuführungsbereich aufnehmen und mitreißen kann.

3. Spray-Applikator nach Anspruch 2, wobei die Klebstoff-Zuführungs-Leitung (18, 124) ein Lumen umfaßt, welches eine Klebstoff-Abgabe-Öffnung (19) hat, welche in der Spray-Kammer an einer Stelle angeordnet ist, an der die Abgabe-Öffnung von dem Gasstrom (26) umgeben ist.

4. Spray-Applikator nach Anspruch 3, wobei die Klebstoff-Abgabeöffnung (19) stromaufwärts vom Eingang (14, 187) der Spray-Kammer positioniert ist, sich nicht durch den Eingang (14, 187) hindurcherstreckt bzw. nicht über diesen hinausragt und in einem spitzen Winkel zu der Richtung des Flusses des Gasstroms (26) angeordnet ist, so daß Klebstoff, welcher aus der Abgabe-Öffnung austritt, durch den Gasfluß vom Lumen weggezogen wird.

5. Spray-Applikator nach Anspruch 1, wobei der Einlaß (12, 302) für Druckgas mit einer Quelle für Druckgas verbindbar ist, welche eine Drucklufrversorgung eines Gebäudes, eine Gasversorgung eines Operationssaals oder ein tragbarer Behälter für Druckgas ist.

6. Spray-Applikator nach Anspruch 5, wobei das Druckgas sauberes, gefiltertes, steriles Inertgas, Luft, Stickstoff, Kohlendioxid, sauerstoffangereicherte Luft oder ein Gas ist, welches eine Medikation oder einen Arbeitsoberflächen-Konditionierer mit sich trägt.

7. Spray-Applikator nach Anspruch 5 oder 6, der weiterhin ein Beabstandungselement (66, 100) umfaßt, welches sich in Sprührichtung erstreckt, um einen erwünschten Abstand zwischen dem Spray-Volumen und der Arbeitsoberfläche aufrecht zu erhalten.

8. Spray-Applikator nach Anspruch 1, 2, 3 oder 4, der weiterhin eine Absaugleitung umfaßt, welche mit einer Absaug-Quelle verbindbar ist und eine Absaugöffnung zur Anwendung der Absaugung an einer zu besprühenden Arbeitsoberfläche, hat.

9. Spray-Applikator nach Anspruch 7, der weiterhin eine Absaugleitung umfaßt, welche mit einer Absaugquelle verbindbar ist, und eine Absaug-Öffnung zur Anwendung der Absaugung an der zu besprühenden Arbeitsoberfläche hat,
wobei die Absaugöffnung in dem Beabstandungselement (66, 100) angeordnet ist.

10. Spray-Applikator nach Anspruch 1, 2, 3 oder 4, wobei die wenigstens zwei Klebstoff-Agenzien dem Spray-Volumen durch eine manuelle Betätigung eines manuellen Stellgliedes zugeführt werden.

11. Spray-Applikator nach Anspruch 10, der weiterhin ein Lumen umfaßt, welches sich von dem Misch-Volumen zum Spray-Volumen erstreckt, wobei das Lumen eine Abgabeöffnung hat, welche in dem Misch-Volumen angeordnet ist, um dem Spray-Volumen den gemischten Klebstoff (21) zuzuführen.

12. Spray-Applikator nach Anspruch 11, wobei das Lumen in das Spray-Volumen hineinragt, um vom Gasstrom (26) vom Gaseinlaß (12, 302) abgetastet bzw. umströmt zu werden.

13. Spray-Applikator nach Anspruch 1. wobei die Freimach-Einrichtung mit der Klebstoff-Zuführungs-Leitung (18, 124) verbindbar ist, um eine retrograde Absaugung auf das Misch-Volumen anzuwenden.

14. Spray-Applikator nach Anspruch 1, 2, 3 oder 4, wobei der Klebstoff ein biologischer Klebstoff ist, welcher für eine chirurgische Verwendung geeignet ist.

15. Spray-Applikator nach Anspruch 14, wobei der Klebstoff ein Fibrin-Klebstoff ist, welcher ein Fibrinogen-Agens und ein Fibrinogen-Aktivator-Agens umfaßt, wobei das Fibrinogen-Agens optional Thrombin ist.

16. Spray-Applikator nach Anspruch 14, der weiterhin wenigstens zwei VorratsBehälter umfaßt, welche als Quelle für die wenigstens zwei Klebstoff-Agenzien dienen.

17. Spray-Applikator nach Anspruch 16, der weiterhin ein äußeres Gehäuse umfaßt und eine Größe, Konfiguration und Anordnung hat, welche dafür geeignet ist, daß der Applikator in bzw. mit einer Hand gehalten und bedient wird.

18. Spray-Applikator nach Anspruch 2, 6, 7 oder 9, wobei die Spray-Kammer eine in Richtung des Flusses des Gasstroms (26) auseinanderlaufende Form hat.

19. Spray-Applikator nach Anspruch 18, wobei die Spray-Kammer so geformt ist, dass sie ein Spray erzeugt, das ein Volumen besetzt, welches in Richtung der Bewegung des Sprays auseinanderläuft.

20. Spray-Applikator nach Anspruch 2, 6, 7 oder 9, wobei die Klebstoff-Agenzien eine koagulierende bzw. gerinnende Klebstoff-Mischung in der Klebstoff-Zuführungs-Leitung vor der Zuführung zu dem Gasstrom (26) umfassen.

21. Spray-Applikator nach Anspruch 2, 6, 7 oder 9, wobei die Klebstoff-Agenzien unter Druck entlang der Zuführungs-Leitung bewegt und in den Gasstrom abgegeben werden.

22. Spray-Applikator nach Anspruch 21, wobei der Applikator Vorratsbehälter für die Klebstoff-Agenzien, ein manuelles Stellglied und eine Ansteuerungseinrichtung umfaßt, um manuelle Kraft anzuwenden und die Klebstoff-Agenzien aus den Vorratsbehältern unter Druck auszulassen.

23. Spray-Applikator nach Anspruch 1, der weiterhin einen Applikator-Körper und eine Spray-Düsenanordnung umfaßt, welche entfernbar am Applikator-Körper angebracht ist, wobei der Applikator-Körper folgendes umfaßt:
a) wenigstens zwei Vorratsbehälter zur Lagerung der wenigstens zwei Klebstoff-Agenzien;
b) wenigstens zwei Leitungen zur separaten Abgabe der wenigstens zwei Klebstoff-Agenzien; und
c) eine manuell betätigbare Abgabeeinheit, um die Klebstoff-Agenzien aus den Vorratsbehältern durch die wenigstens zwei Leitungen freizusetzen;
wobei die Spray-Düsenanordnung folgendes umfaßt:
d) wenigstens zwei Mündungen bzw. Anschlüsse, welche die Klebstoff-Agenzien aufnehmen und mit den wenigstens zwei Leitungen verbindbar sind;
e) eine Misch-Kammer zur Aufnahme und zum Vermischen der wenigstens zwei Klebstoff-Agenzien von den wenigstens zwei Anschlüssen, welche den Klebstoff aufnehmen, um den gemischten Klebstoff bereitzustellen;
f) das Spray-Volumen;
g) eine Zuführungs-Leitung, um den gemischten Klebstoff aus dem Misch-Volumen aufzunehmen und um den gemischten Klebstoff dem Spray-Volumen zuzuführen; und
h) den Einlaß (12, 302) für Druckgas;
und wobei die Zuführungs-Leitung sich in den Weg des Gasstroms (26) hineinerstreckt, um den gemischten Klebstoff dem Gasstrom (26) zuzuführen und das Spray aus gemischten Klebstoff-Tröpfchen zu erzeugen.

24. Spray-Applikator nach Anspruch 23, wobei die Spray-Düsenanordnung ein Beabstandungselement (66, 100) umfaßt, welche sich in Sprührichtung erstreckt, um einen gewünschten Abstand zwischen dem Spray-Volumen und einer Arbeitsoberfläche aufrecht zu erhalten.

25. Spray-Applikator nach Anspruch 23, wobei die Spray-Düsenanordnung eine Absaugleitung (18, 124) umfaßt, welche mit einer Absaugquelle verbindbar ist und eine Absaugöffnung hat, um die Absaugung an einer zu besprühenden Arbeitsoberfläche anzuwenden, wobei die Absaugöffnung in dem Beabstandungselement (66, 100) angeordnet ist.

26. Spray-Applikator nach Anspruch 23, wobei die Freimach-Einrichtung mit dem Misch-Volumen verbindbar ist, um eine retrograde Absaugung anzuwenden, um das Misch-Volumen und die Zuführungs-Leitung freizumachen.

27. Spray-Applikator nach einem der Ansprüche 23 bis 26, welcher dazu eingerichtet ist, in bzw. mit einer Hand gehalten und bedient zu werden.

28. Spray-Düsenanordnung zur Verwendung in dem Spray-Applikator nach Anspruch 23, wobei die Spray-Düsenanordnung folgendes umfaßt:
d) wenigstens zwei Mündungen bzw. Anschlüsse, welche die Klebstoff-Agenzien aufnehmen und mit den wenigstens zwei Leitungen verbindbar sind;
e) eine Misch-Kammer zur Aufnahme und zum Vermischen der wenigstens zwei Klebstoff-Agenzien von den wenigstens zwei Anschlüssen, welche den Klebstoff aufnehmen, um den gemischten Klebstoff bereitzustellen;
f) das Spray-Volumen;
g) eine Zuführungs-Leitung, um den gemischten Klebstoff aus dem Misch-Volumen aufzunehmen und um den gemischten Klebstoff dem Spray-Volumen zuzuführen;
h) den Einlaß (12, 302) für Druckgas; und
i) eine Freimach-Einrichtung, die eingerichtet ist, um durch die Anwendung der Absaugung verklumptes Klebstoff-Material aus der Verteilungs-Leitung zu räumen;
wobei die Zuführungs-Leitung sich in den Weg des Gasstroms (26) hineinerstreckt, um den gemischten Klebstoff dem Gasstrom (26) zuzuführen und das Spray aus gemischten Klebstoff-Tröpfchen zu erzeugen.

29. Spray-Düsenanordnung nach Anspruch 28, welche weiterhin ein Beabstandungselement (66, 100) umfaßt, welches sich in Sprührichtung erstreckt, um einen gewünschten Abstand zwischen dem Sprayvolumen und einer Arbeitsoberfläche aufrecht zu erhalten.

30. Spray-Düsenanordnung nach Anspruch 29, welche weiterhin eine Absaugleitung umfaßt, welche mit einer Absaugquelle verbindbar ist und eine Absaugöffnung hat, um die Absaugung an einer zu besprühenden Arbeitsoberfläche anzuwenden, wobei die Absaugöffnung in dem Beabstandungselement (66, 100) angeordnet ist.

31. Spray-Düsenanordnung nach einem der Ansprüche 28 bis 30, wobei die Feimach-Einrichtung mit dem Misch-Volumen verbindbar ist, um eine retrograde Absaugung anzuwenden, um das Misch-Volumen und die Zuführungs-Leitung freizumachen.

## Revendications

1. Applicateur de pulvérisation pour pulvériser un agent de scellement liquide comprenant au moins deux agents de scellement capables de coaguler lorsqu'ils sont mélangés l'un avec l'autre, l'applicateur comprenant :
a) un volume de pulvérisation ;
b) une voie de passage de distribution d'agent de scellement (18, 124) s'étendant à partir de sources individuelles des agents de scellement respectifs jusqu'au volume de pulvérisation pour la distribution des au moins deux agents de scellement pour fournir un agent de scellement mélangé (21) et pour distribuer l'agent de scellement mélangé au volume de pulvérisation ;
c) une entrée de gaz sous pression (12, 302) adaptée pour admettre un courant gazeux (26) dans le volume de pulvérisation ;
dans lequel la voie de passage de distribution d'agent de scellement (18, 124) est adaptée pour distribuer l'agent de scellement mélangé au courant gazeux (26) de sorte qu'une pulvérisation contenant l'agent de scellement mélangé est générée dans le volume de pulvérisation ; et
d) un dispositif de nettoyage adapté pour nettoyer un matériau de scellement accumulé de la voie de passage de distribution (18, 124) en appliquant une aspiration.

2. Applicateur de pulvérisation selon la revendication 1, comprenant en outre une chambre de pulvérisation définissant le volume de pulvérisation, la chambre de pulvérisation ayant une embouchure (14, 187), ayant l'entrée de gaz sous pression (12, 302) disposée de façon éloignée de l'embouchure (14, 187) et ayant une région de distribution d'agent de scellement disposée entre l'embouchure (14, 187) et la source de gaz moyennant quoi le courant gazeux (26) passe à travers le volume de pulvérisation à partir de la source de gaz jusqu'à l'embouchure (14, 187) et peut recevoir et entraîner les agents de scellement dans la région de distribution.

3. Applicateur de pulvérisation selon la revendication 2, dans lequel la voie de passage de distribution d'agent de scellement (18, 124) comprend une lumière ayant une ouverture de distribution d'agent de scellement (19) disposée dans la chambre de pulvérisation dans un emplacement où l'ouverture de distribution est entourée par le courant gazeux (26).

4. Applicateur de pulvérisation selon la revendication 3, dans lequel l'ouverture de distribution d'agent de scellement (19) est positionnée en amont de l'embouchure (14, 187) de la chambre de pulvérisation, ne fait pas saillie à travers l'embouchure (14, 187) et est disposée à un angle aigu par rapport à la direction d'écoulement du courant gazeux (26) de sorte que l'agent de scellement émergeant de l'ouverture de distribution est éloigné de la lumière par l'écoulement gazeux.

5. Applicateur de pulvérisation selon la revendication 1, dans lequel l'entrée de gaz sous pression (12, 302) peut être raccordée à une source de gaz sous pression étant une alimentation en air comprimé d'un bâtiment, une alimentation en gaz d'une salle d'opération chirurgicale ou un récipient portable de gaz comprimé.

6. Applicateur de pulvérisation selon la revendication 5, dans lequel le gaz sous pression est un gaz inerte, de l'air, de l'azote, du dioxyde de carbone, de l'air enrichi d'oxygène propre, filtré, stérile, ou un gaz transportant un médicament ou un conditionneur de surface de travail.

7. Applicateur de pulvérisation selon la revendication 5 ou 6, comprenant en outre un élément d'entretoise (66, 100) s'étendant dans la direction de pulvérisation pour maintenir une distance souhaitée entre le volume de pulvérisation et une surface de travail.

8. Applicateur de pulvérisation selon la revendication 1, 2, 3 ou 4, comprenant en outre une voie de passage d'aspiration pouvant être raccordée à une source d'aspiration et ayant une ouverture d'aspiration pour appliquer une aspiration sur une surface de travail destinée à être pulvérisée.

9. Applicateur de pulvérisation selon la revendication 7, comprenant en outre une voie de passage d'aspiration pouvant être raccordée à une source d'aspiration et ayant une ouverture d'aspiration pour appliquer une aspiration sur la surface de travail destinée à être pulvérisée, dans lequel l'ouverture d'aspiration est située dans l'élément d'entretoise (66, 100).

10. Applicateur de pulvérisation selon la revendication 1, 2, 3 ou 4, dans lequel les au moins deux agents de scellement sont distribués au volume de pulvérisation par l'intermédiaire de l'opération manuelle d'un actionneur manuel.

11. Applicateur de pulvérisation selon la revendication 10, comprenant en outre une lumière s'étendant à partir du volume de mélange jusqu'au volume de pulvérisation, la lumière ayant une ouverture de distribution disposée dans le volume de mélange pour distribuer l'agent de scellement mélangé (21) au volume de pulvérisation.

12. Applicateur de pulvérisation selon la revendication 11, dans lequel la lumière fait saillie dans le volume de pulvérisation destiné à être balayé par le courant gazeux (26) à partir de l'entrée de gaz (12, 302).

13. Applicateur de pulvérisation selon la revendication 1, dans lequel le dispositif de nettoyage peut être raccordé à la voie de passage de distribution d'agent de scellement (18, 124) pour appliquer une aspiration rétrograde sur le volume de mélange.

14. Applicateur de pulvérisation selon la revendication 1, 2, 3 ou 4, dans lequel l'agent de scellement est un agent de scellement biologique approprié pour une utilisation chirurgicale.

15. Applicateur de pulvérisation selon la revendication 14, dans lequel l'agent de scellement est un agent de scellement en fibrine comprenant un agent fibrinogène et un agent de fibrinoformation, ledit agent fibrinogène étant facultativement de la thrombine.

16. Applicateur de pulvérisation selon la revendication 14, comprenant en outre au moins deux réservoirs servant de sources pour les au moins deux agents de scellement.

17. Applicateur de pulvérisation selon la revendication 16, comprenant en outre un logement extérieur et ayant une dimension, configuration et un agencement approprié pour que l'applicateur soit saisi et actionné dans une main.

18. Applicateur de pulvérisation selon la revendication 2, 6, 7 ou 9, dans lequel la chambre de pulvérisation a une forme divergeant dans la direction d'écoulement du courant gazeux (26).

19. Applicateur de pulvérisation selon la revendication 18, dans lequel la chambre de pulvérisation est formée pour générer une pulvérisation occupant un volume qui diverge dans la direction de déplacement de la pulvérisation.

20. Applicateur de pulvérisation selon la revendication 2, 6, 7 ou 9, dans lequel les agents de scellement comprennent un mélange d'agent de scellement coagulant dans la voie de passage de distribution d'agent de scellement avant la distribution au courant gazeux (26).

21. Applicateur de pulvérisation selon la revendication 2, 6, 7 ou 9, dans lequel les agents de scellement sont déplacés le long de la voie de passage de distribution sous pression et libérés dans le courant gazeux.

22. Applicateur de pulvérisation selon la revendication 21, dans lequel l'applicateur comprend des réservoirs pour les agents de scellement, un actionneur manuel et un mécanisme d'entraînement pour appliquer une force manuelle et entraîner les agents de scellement hors des réservoirs sous pression.

23. Applicateur de pulvérisation selon la revendication 1, comprenant en outre un corps d'applicateur et un ensemble de buse de pulvérisation attaché de façon amovible au corps d'applicateur de pulvérisation, dans lequel le corps d'applicateur a :
a) au moins deux réservoirs pour stocker respectivement les au moins deux agents de scellement ;
b) au moins deux conduits pour distribuer séparément les au moins deux agents de scellement ; et
c) un mécanisme de distribution actionnable manuellement pour décharger les agents de scellement à partir des réservoirs à travers les au moins deux conduits ;
dans lequel l'ensemble de buse de pulvérisation a :
d) au moins deux orifices de réception d'agent de scellement pouvant être raccordés aux au moins deux conduits ;
e) une chambre de mélange pour recevoir et mélanger les au moins deux agents de scellement à partir des au moins deux orifices de réception d'agent de scellement pour fournir l'agent de scellement mélangé ;
f) un volume de pulvérisation ;
g) un conduit de distribution pour recevoir un agent de scellement mélangé provenant du volume de mélange et distribuer l'agent de scellement mélangé au volume de pulvérisation ; et
h) l'entrée de gaz sous pression (12, 302) ;
et dans lequel le conduit de distribution s'étend dans le passage du courant gazeux (26) pour distribuer l'agent de scellement mélangé au courant gazeux (26) et générer la pulvérisation de gouttelettes d'agent de scellement mélangé.

24. Applicateur de pulvérisation selon la revendication 23, dans lequel l'ensemble de buse comprend un élément d'entretoise (66, 100) s'étendant dans la direction de pulvérisation pour maintenir une distance souhaitée entre le volume de pulvérisation et une surface de travail.

25. Applicateur de pulvérisation selon la revendication 24, dans lequel l'ensemble de buse comprend une voie de passage d'aspiration (18, 124) pouvant être raccordée à une source d'aspiration et ayant une ouverture d'aspiration pour appliquer une aspiration à une surface de travail destinée à être pulvérisée, dans lequel l'ouverture d'aspiration est située dans l'élément d'entretoise (66, 100).

26. Applicateur de pulvérisation selon la revendication 23, dans lequel le dispositif de nettoyage peut être raccordé au volume de mélange pour appliquer une aspiration rétrograde pour nettoyer le volume de mélange et le conduit de distribution.

27. Applicateur de pulvérisation selon les revendications 23 à 26, qui est adapté pour être saisi et actionné dans une main.

28. Ensemble de buse de pulvérisation destiné à être utilisé dans l'applicateur de pulvérisation de la revendication 23 ;
dans lequel l'ensemble de buse de pulvérisation comprend :
d) au moins deux orifices de réception d'agent de scellement pouvant être raccordés aux au moins deux conduits ;
e) une chambre de mélange pour recevoir et mélanger les au moins deux agents de scellement à partir des au moins deux orifices de réception d'agent de scellement pour fournir l'agent de scellement mélangé ;
f) un volume de pulvérisation ;
g) un conduit de distribution pour recevoir l'agent de scellement mélangé provenant du volume de mélange et pour distribuer l'agent de scellement mélangé au volume de pulvérisation ;
h) l'entrée de gaz sous pression (12, 302) ; et
i) un dispositif de nettoyage adapté pour nettoyer le matériau de scellement accumulé de la voie de passage de distribution en appliquant une aspiration ;
dans lequel le conduit de distribution s'étend dans le passage du courant gazeux (26) pour distribuer l'agent de scellement mélangé au courant gazeux (26) pour générer une pulvérisation de gouttelettes d'agent de scellement mélangé.

29. Ensemble de buse de pulvérisation selon la revendication 28, comprenant en outre un élément d'entretoise (66, 100) s'étendant dans la direction de pulvérisation pour maintenir une distance souhaitée entre le volume de pulvérisation et une surface de travail.

30. Ensemble de buse de pulvérisation selon la revendication 29, comprenant en outre une voie de passage d'aspiration pouvant être raccordée à une source d'aspiration et ayant une ouverture d'aspiration pour appliquer une aspiration sur une surface de travail destinée à être pulvérisée, dans lequel l'ouverture d'aspiration est située dans l'élément d'entretoise (66, 100).

31. Ensemble de buse de pulvérisation selon l'une quelconque des revendications 28 à 30, dans lequel le dispositif de nettoyage peut être raccordé au volume de mélange pour appliquer une aspiration rétrograde pour nettoyer le volume de mélange et le conduit de distribution.
